# EUROPEAN PATENT SPECIFICATION

(11) **EP 4 635 541 B1**
(45) Date of publication and mention of the grant of the patent: **29.04.2026**
(21) Application number: 24182488.7
(22) Date of filing: 17.06.2024
(51) Int. Cl.: A61M 16/00

(54) **PORTABLE RESPIRATORY SYSTEM**
TRAGBARES BEATMUNGSSYSTEM
SYSTÈME RESPIRATOIRE PORTABLE

(30) Priority: 15.04.2024 TW 113114042
(43) Date of publication of application: 22.10.2025
(73) Proprietor: Wellell Inc., New Taipei City 23679 (TW)
(72) Inventor: Lin, Chun-Yen, New Taipei City (TW); Chen, Kuan-Chih, New Taipei City (TW)
(74) Representative: Schwerbrock, Florian

(56) References cited:
- US-A1- 2023 398 318
- US-B2- 11 712 529

## Description

### BACKGROUND OF THE INVENTION

### FIELD OF THE INVENTION

The present invention relates to portable respiratory systems and, more particularly, to a portable respiratory system having a flow sensor.

### DESCRIPTION OF THE PRIOR ART

A portable respiratory system is capable of providing a patient with a required amount of airflow under a corresponding setting of the system according to user requirements, thereby providing effective treatment. According to a set amount of flow, the portable respiratory system performs corresponding control and regulation, for example, regulating the intensity or pressure of airflow to improve comfort. For certain patients, an overly high pressure can cause discomfort, and an inadequately low pressure can fail to effectively maintain smooth breathing and hence unable to provide effective treatment or even fail to effectively preventing an occurrence of a respiratory arrest.

Thus, whether a portable respiratory system is able to accurately control an airflow is closely related to therapeutic effectiveness and wearing comfort, and further affects energy utilization efficiency of the system.

A typical household respiratory system, such as that disclosed in US Patent Application 11712529B2, comprises a housing, a partition element, elastic components, rigid component, a blower and other structural elements. The partition element divides the interior of the housing into an electrical component chamber and a fluid chamber. The electrical component chamber accommodates a circuit board and flow sensors, whilst the fluid chamber houses the blower, elastic components encasing the blower, and rigid component forming the flow passage. The flow sensor measures the flow rate within the passage. Since household respiratory systems are typically used at a fixed location, the requirements for portable use during travel have not been considered. Consequently, these household respiratory systems are bulky, with internal components arranged with considerable flexibility, and often featuring extended flow passages to reduce noise levels. However, such household respiratory systems fail to achieve compact and lightweight configurations, making it difficult for users to conveniently and comfortably use them whilst travelling.

### SUMMARY OF THE INVENTION

It is an object of the present invention to improve therapeutic effectiveness and comfort of a miniature and lightweight portable respiratory system.

It is another object of the present invention to provide a portable respiratory system having a flow detection function.

To achieve the above and other objects, the present invention provides a portable respiratory system including a housing, a divider, a flow sensor, an elastic member, a rigid member, a blower and a flow limiter. The divider is disposed in the housing and partitions an internal space of the housing into an upper chamber and a lower chamber. The flow sensor is disposed in the upper chamber. The elastic member is disposed in the lower chamber. The rigid member is fixed in the lower chamber by the elastic member and the housing, and defines an air outlet flow channel, a lateral flow channel, a transmission flow channel and an air outlet flow channel, wherein the lateral flow channel is connected between the air inlet flow channel and the transmission flow channel. The blower is disposed in the elastic member, and is operable to receive an airflow from the transmission flow channel to generate a positive pressure airflow and transmit the airflow to the air outlet flow channel. The flow limiter is disposed in the lateral flow channel, and includes a plurality of pipes which extend along the lateral flow channel. The rigid member further includes a first detection passage and a second detection passage. The first detection passage is located in an upstream of the flow limiter, the second detection passage is located in a downstream of the flow limiter, and the first detection passage and the second detection passage are individually connected to the flow sensor. The flow limiter is disposed below the air outlet flow channel of the rigid member.

According to some embodiments of the present invention, each of the first detection passage and the second detection passage has a lower opening, and the two lower openings are higher than an upper edge of a highest one of the pipes.

According to some embodiments of the present invention, the lower opening of the first detection passage and top portions of the pipes define a first buffer space in between, the lower opening of the second detection passage and the top portions of the pipes define a second buffer space in between, and airflow speeds in the first buffer space and the second buffer space are lower than an airflow speed at an opening of each of the pipes.

According to some embodiments of the present invention, the first buffer space and the second buffer space are in non-symmetrical arrangement in between with respect to a central axis of the air outlet flow channel.

According to some embodiments of the present invention, each of the pipes extends from right below a central axis of the air outlet flow channel in a direction away from the air inlet flow channel by a first length and extends in a direction toward the air inlet flow channel by a second length, wherein the first length is greater than the second length.

According to some embodiments of the present invention, a vertical distance between the two lower openings and the upper edge of the highest one of the pipes is a first distance, which is greater than or equal to a half of an inner diameter of the pipe.

According to some embodiments of the present invention, the first detection passage and the second detection passage protrude downward into the lateral flow channel.

According to some embodiments of the present invention, the flow limiter includes a sealing member. The sealing member covers on an outside of the pipes so as to fix the pipes, and the sealing member and an outer wall of the air outlet flow channel form airtightness in between.

According to some embodiments of the present invention, the flow limiter is detachably disposed in the lateral flow channel.

According to some embodiments of the present invention, the elastic member includes an upper wind guide cover and a lower shock absorbing cover. The upper wind guide cover and the blower define an input flow channel, the input flow channel is connected to the transmission flow channel to guide an airflow to flow into the blower, and the upper wind guide cover has a plurality of flow guide protrusions protruding downward.

According to some embodiments of the present invention, the upper wind guide cover has an exhaust hole which is operable for a heat exhaust pipe of the blower to dissipate heat energy.

According to some embodiments of the present invention, the rigid member has a plurality of protruding flow guide plates and a spacing plate at the air inlet flow channel. The flow guide plates separate a front section of the air inlet flow channel into a plurality of air inlet channels, the spacing plate is disposed at a middle section of the air inlet flow channel, and a bottom edge of the spacing plate is closer to a bottom portion of the housing than bottom edges of the flow guide plates.

According to some embodiments of the present invention, the lower shock absorbing cover has a positioning wall and a water blocking wall protruding upward. The positioning wall covers at least a portion of the blower and has a notch to receive an electrical portion of the blower, and the water blocking wall is disposed on the periphery of the notch.

According to some embodiments of the present invention, the portable respiratory system further includes a circuit board, a light emitting element, a button element, a shade hood and a light guide column. The circuit board is disposed in the upper chamber, the light emitting element is disposed on the circuit board, the button element is disposed on the circuit board, the shade hood covers the light emitting element and includes a through opening, and the light guide column is disposed in the through opening to guide light.

According to some embodiments of the present invention, the through opening has a first section and a second section. The first section is operable to receive the light guide column, the second section is operable to cover the light emitting element, and an inner diameter of the second section is greater than an inner diameter of the first section.

According to some embodiments of the present invention, the shade hood further includes a protruding unit, and a lower portion of the protruding unit corresponds to the button element on the circuit board. A press member is disposed on an outer surface of the housing, and a top surface of the protruding unit has a slot for matching with a bottom surface structure of the corresponding press member.

According to some embodiments of the present invention, the slot is a cross positioning slot, and the bottom surface structure is a cross positioning rib matching with the cross positioning slot.

According to some embodiments of the present invention, a bottom surface of the press member has a support wall. The support wall is disposed around the bottom surface structure to enhance stability when the press member is pressed downward.

According to some embodiments of the present invention, the bottom surface of the press member has a stop portion, and the shade hood has thereon a recess for correspondingly accommodating the stop portion.

Accordingly, the portable respiratory system in the embodiments of the present invention features miniaturization, lightweight and easy portability, and forms an arrangement of an accommodation space and flow channels by structures of the divider, the elastic member, the rigid member and the flow limiter, further achieving a compact design. Meanwhile, the portable respiratory system has a flow monitoring function, and is able to regulate an amount of airflow or perform control of different air supply modes according to user requirements, thus improving comfort and experience of user of the respiratory system and at the same time promoting implementation of better therapeutic effects.

### BRIEF DESCRIPTION OF THE DRAWINGS

FIG. 1 is a perspective schematic diagram of a portable respiratory system according to an embodiment of the present invention;
FIG. 2 is an exploded schematic diagram of the portable respiratory system in FIG. 1;
FIG. 3 is a partial schematic diagram of the portable respiratory system of the embodiment in FIG. 1;
FIG. 4 is a cross-sectional schematic diagram along the section line A-A in FIG. 1;
FIG. 5(a) is a perspective schematic diagram of a rigid member in a portable respiratory system according to this embodiment of the present invention;
FIG. 5(b) is a side schematic diagram of a rigid member in a portable respiratory system according to this embodiment of the present invention;
FIG. 6 is a cross-sectional schematic diagram along the section line B-B in FIG. 1;
FIG. 7 is a perspective schematic diagram of an elastic member in a portable respiratory system according to this embodiment of the present invention;
FIG. 8 is a partial exploded schematic diagram of a flow limiter, a rigid member, a lower shock absorbing cover and a lower shell of a portable respiratory system according to an embodiment of the present invention;
FIG. 9 is a schematic diagram of a blower and a lower shock absorbing cover in a portable respiratory system according to an embodiment of the present invention;
FIG. 10 is a schematic diagram of a blower and an upper wind guide cover in a portable respiratory system according to an embodiment of the present invention;
FIG. 11 is a schematic diagram of a blower and an upper wind guide cover in a portable respiratory system according to another embodiment of the present invention;
FIG. 12 is a partial exploded schematic diagram of a portable respiratory system according to an embodiment of the present invention;
FIG. 13 is a cross-sectional schematic diagram of a portion of FIG. 12;
FIG. 14 is a schematic diagram of a shade hood in a portable respiratory system according to an embodiment of the present invention; and
FIG. 15 is a schematic diagram of an upper shell having a press member in a portable respiratory system according to an embodiment of the present invention.

### DETAILED DESCRIPTION OF THE EMBODIMENTS

Objectives, features, and advantages of the present disclosure are hereunder illustrated with specific embodiments, depicted with drawings, and described below.

In the disclosure, descriptive terms such as "include, comprise, have" or other similar terms are not for merely limiting the essential elements listed in the disclosure, but can include other elements that are not explicitly listed and are however usually inherent in the components, structures, devices, portions, sections or regions.

In the disclosure, the terms similar to ordinals such as "first" or "second" described are for distinguishing or referring to associated identical or similar components or structures, and do not necessarily imply the orders of these components, structures, devices, portions, sections or regions in a spatial aspect. It should be understood that, in some situations or configurations, the ordinal terms could be interchangeably used without affecting the implementation of the present invention.

In the disclosure, descriptive terms such as "a" or "one" are used to describe the components, structures, devices, portions, sections or regions, and are for illustration purposes and providing generic meaning to the scope of the present invention. Therefore, unless otherwise explicitly specified, such description should be understood as including one or at least one, and a singular number also includes a plural number.

In an embodiment of the present invention, a portable respiratory system is exemplified by a miniature, lightweight and portable continuous positive airway pressure (CPAP) machine. A portable respiratory system is applicable in response to an environment of extensive requirements during traveling, working or camping for a user. Thus, for the purposes of miniaturization, lightweight and easy portability, element configurations and flow channel arrangements are limited to characteristics of a limited internal space and compactness.

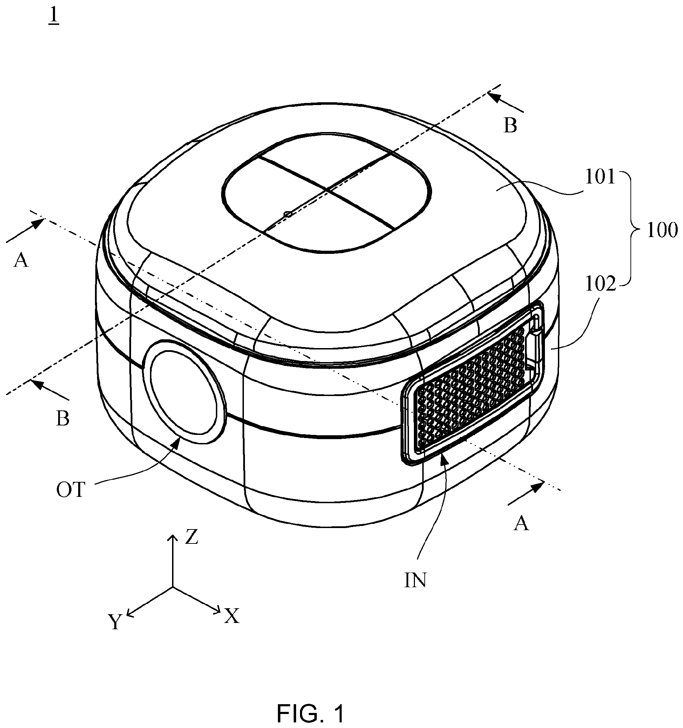

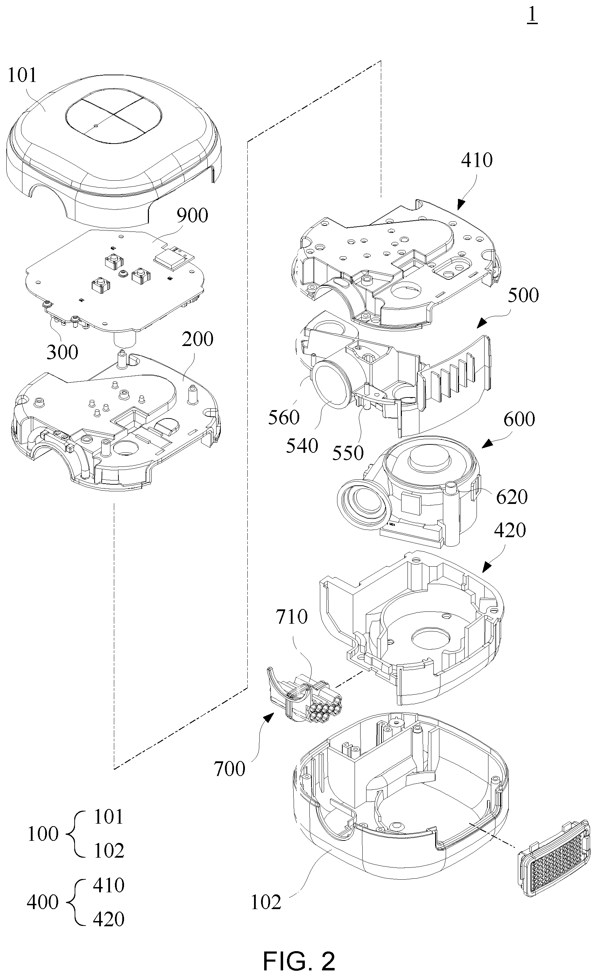

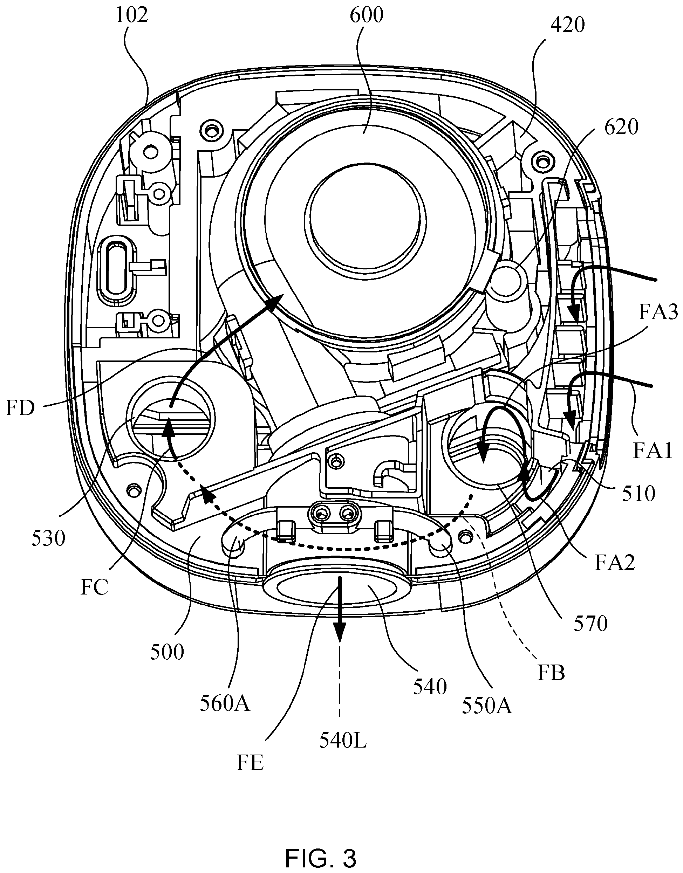

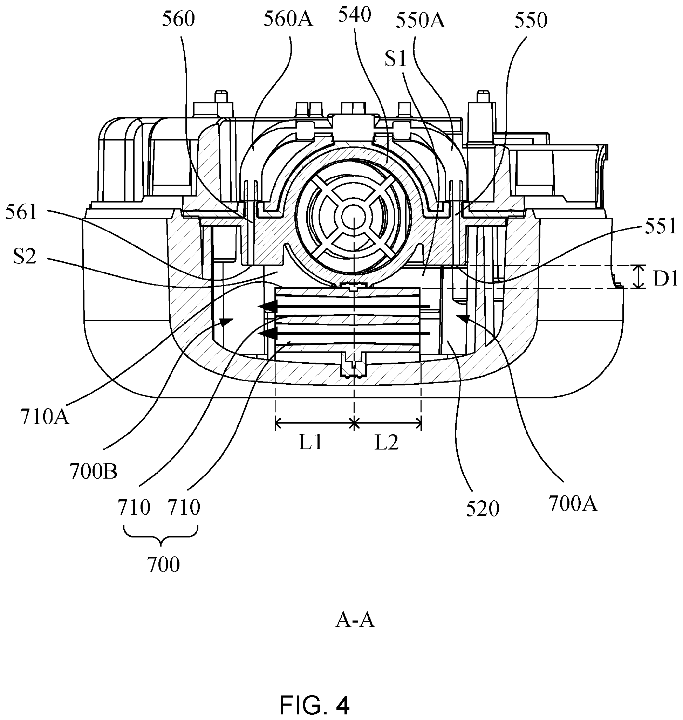

Referring to FIG. 1 to FIG. 4, FIG. 1 shows a perspective schematic diagram of a portable respiratory system according to an embodiment of the present invention, FIG. 2 shows an exploded schematic diagram of the portable respiratory system in FIG. 1, FIG. 3 shows a partial schematic diagram of the portable respiratory system of the embodiment in FIG. 1, and FIG. 4 shows a cross-sectional schematic diagram along the section line A-A in FIG. 1.

As shown in FIG. 1, a portable respiratory system 1 of this embodiment includes a housing 100. The housing 100 can include an upper shell 101 and a lower shell 102. The upper shell 101 and the lower shell 102, when assembled, can define an internal space and a plurality of externally communicating windows, for example, an air inlet IN and an air outlet OT.

As shown in FIG. 2, the portable respiratory system 1 of this embodiment further includes a circuit board 900, a divider 200, an elastic member 400, a rigid member 500, a blower 600 and a flow limiter 700.

The divider 200 is disposed in the housing 100, and partitions the internal space of the housing 100 into an upper chamber and a lower chamber. The upper chamber can be used for disposing circuits and electrical components so as to correspond to function press members for user operation and control on the upper shell 101. For example, a circuit board 900 can be disposed in the upper chamber, and the flow sensor 300 can be disposed on the circuit board 900 to detect an amount of flow of airflow. The lower chamber can be used for disposing components for generating a positive pressure airflow, and these components are for constructing a path channel for an airflow to flow through. For example, the elastic member 400, the rigid member 500, the blower 600 and the flow limiter 700 are disposed in the lower chamber.

The elastic member 400 sandwiches the blower 600 therein; the elastic member 400 has a portion located between the divider 200 and the blower 600 and has a portion located between the blower 600 and the housing 100, so as to position the blower 600. Moreover, the elastic member 400 separates the blower 600 from the rigid divider 200 and the housing 100, further providing shock absorption and noise reduction effects. The rigid member 500 is fixed in the lower chamber by the elastic member 400 and the housing 100, with details of the fixing means to be described shortly.

As shown in FIG. 3, the rigid member 500 is operable to define flow channels of the portable respiratory system 1, wherein the flow channels include an air inlet flow channel 510, a lateral flow channel 520 (as shown in FIG. 4), a transmission flow channel 530 and an air outlet flow channel 540, wherein the lateral flow channel 520 is connected between the air inlet flow channel 510 and the transmission flow channel 530. The blower 600 is disposed in the elastic member 400 and is operable to generate a positive pressure airflow.

In this embodiment, a path through which an airflow flows is as follows. First of all, air enters the housing 100 through the air inlet IN of the housing 100, and enters an air inlet of the blower 600 along the air inlet flow channel 510, the lateral flow channel 520 and the transmission flow channel 530. The blower 600 receives the airflow from the transmission flow channel 530 and generates a positive pressure airflow, and the pressurized airflow is discharged from an air outlet of the blower 600, enters the air outlet flow channel 540 and leaves from the air outlet OT of the housing 100, and is transported via a connected pipeline to a breathing mask (not shown) worn by a user. Further, referring to FIG. 3, in this embodiment, the airflow enters the air inlet flow channel 510 from the air inlet IN, as shown by an airflow path FA1; then, the airflow is lifted, as shown by an airflow path FA2; the airflow then descends and enters the lateral flow channel 520, as shown by an airflow path FA3; next, the airflow flows in the lateral flow channel 520, as shown by an airflow path FB; then, the airflow is lifted in the transmission flow channel 530, as shown by an airflow path FC; next, the airflow flowing out from the transmission flow channel 530 flows to the blower 600, as shown by an airflow path FD; eventually, the airflow flowing out from the blower 600 is output from the air outlet flow channel 540, as shown by an airflow path FE.

As shown in FIG. 4, the flow limiter 700 is disposed in the lateral flow channel 520, and includes multiple pipes 710 extending along the lateral flow channel 520. The flow limiter 700 is operable to cause the airflow passing through the pipes 710 to form a laminar flow and accordingly generate a pressure drop, for the flow sensor 300 to measure the flow rate of airflow.

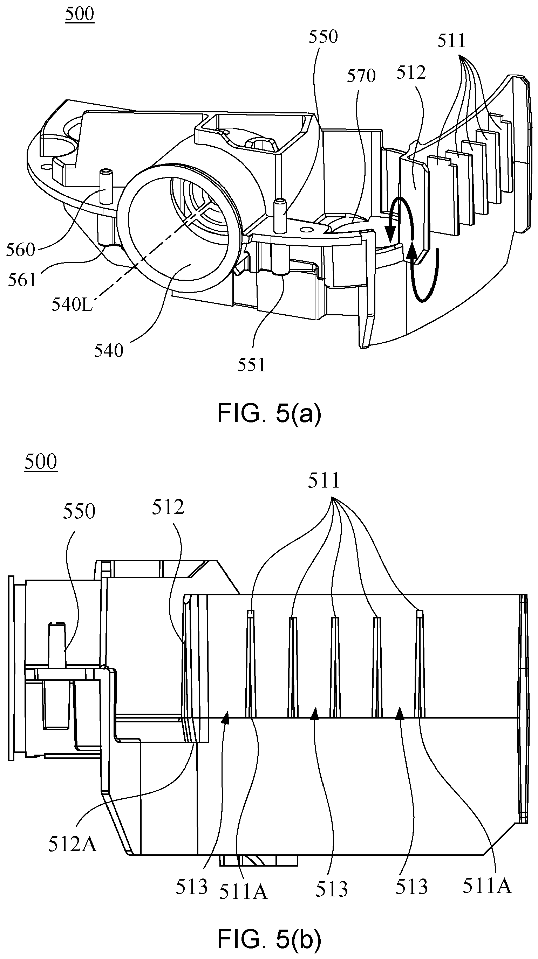

Referring to FIG. 4, FIG. 5(a) and FIG. 5(b), FIG. 5(a) shows a perspective schematic diagram of a rigid member in a portable respiratory system according to this embodiment of the present invention, and FIG. 5(b) shows a side schematic diagram of a rigid member in a portable respiratory system according to this embodiment of the present invention.

The rigid member 500 further includes a first detection passage 550 and a second detection passage 560. The first detection passage 550 is located in an upstream 700A of the flow limiter 700, the second detection passage 560 is located in a downstream 700B of the flow limiter 700, and the first detection passage 550 and the second detection passage 560 are individually connected to the flow sensor 300. The flow sensor 300 obtains a pressure value of each of the upstream 700A and the downstream 700B to thereby calculate the flow rate. In this embodiment, the first detection passage 550 and the second detection passage 560 are preshaped through holes or tubular structures on the rigid member 500, and are connected to the flow sensor 300 by flexible pipes 550A and 560A, respectively. However, the present invention is not limited to the examples above. Alternatively, the first detection passage 550 and the second detection passage 560 can also be integrally formed passages with a sufficient length on the rigid member 500 and be directly connected to a mounting position of a sensor head of the flow sensor 300.

Accordingly, with the arrangement of the first detection passage 550 and the second detection passage 560 of the rigid member 500 in the portable respiratory system of this embodiment of the present invention, the flow sensor 300 located in the upper chamber is allowed to measure the pressures of the upstream 700A and the downstream 700B of the flow limiter 700 to obtain the amount of flow of a fluid in the lateral flow channel 520, hence also providing a flow monitoring function while achieving miniaturization and lightweight of the portable respiratory system. On the basis of the flow monitoring function, the portable respiratory system can more effectively regulate the amount of airflow according to different user requirements or different users, or provide multiple airflow control modes, further improving comfort and experience of user. In addition, better therapeutic effects can also be achieved on the basis of such accurate control over the amount of flow.

Referring to FIG. 4 to FIG. 6, FIG. 6 shows a cross-sectional schematic diagram along the section line B-B in FIG. 1.

As shown in FIG. 4 to FIG. 6, the rigid member 500 has the first detection passage 550 and the second detection passage 560. The first detection passage 550 and the second detection passage 560 can protrude downward into the lateral flow channel 520. The first detection passage 550 and the second detection passage 560 can include lower openings 551 and 561, respectively, and the lower opening 551 of the first detection passage 550 and the lower opening 561 of the second detection passage 560 are higher than an upper edge 710A of a highest one of the pipes 710 of the flow limiter 700.

In terms of height, either of the lower opening 551 and the lower opening 561 is located from the upper edge 710A of the highest pipe 710 by a vertical distance, but is not flushed or aligned with the upper edge 710A of the highest pipe 710. As such, the lower opening 551 of the first detection passage 550 and the lower opening 561 of the second detection passage 560 are not located on an airflow path with a faster flow speed and instability, and so the airflow received by the flow sensor 300 is also more stable, thereby enhancing the stability and accuracy of the amount of flow measured by the portable respiratory system.

As an example, the vertical distance of the two lower openings 551 and 561 from the upper edge 710A of the highest pipe 710 is a first distance D1 (FIG. 4), which is greater than or equal to a half of an inner diameter of the pipe 710. Assuming that the length and the width of the overall appearance of the portable respiratory system 1 are not greater than 95 mm and the height is not greater than 65 mm, the first distance D1 can be designed to be 3 to 15 mm or greater than 15 mm.

For a compact arrangement to reduce the overall volume of the portable respiratory system 1, the flow limiter 700 can be disposed below the air outlet flow channel 540 of the rigid member 500, and the first detection passage 550 and the second detection passage 560 extend on two sides of the air outlet flow channel 540. The lower opening 551 of the first detection passage 550 and top portions of the pipes 710 define a first buffer space S1 in between, and the lower opening 561 of the second detection passage 560 and the top portions of the pipes 710 define a second buffer space S2 in between. An airflow speed in the first buffer space S1 is lower an airflow speed at a pipe opening in the upstream 700A of each pipe 710. An airflow speed in the second buffer space S2 is lower an airflow speed at a pipe opening in the downstream 700B of each pipe 710.

Again referring to FIG. 4, the first buffer space S1 and the second buffer space S2 are in a non-symmetrical arrangement in between with respect to a central axis 540L of the air outlet flow channel 540. In response to the flow field distribution of the upstream 700A and the downstream 700B of the flow limiter 700, the shape of the first buffer space S1 and the shape of the second buffer space S2 are not mirrored, so as to achieve an optimal arrangement.

For example, each pipe 710 can extend from right below the central axis 540L of the air outlet flow channel 540 in a direction away from the air inlet flow channel 510 by a first length L1, and extend in a direction toward the air inlet flow channel 510 by a second length L2, wherein the first length L1 is greater than the second length L2. Thus, a shortest distance between the lower opening 561 of the second detection passage 560 and the top portions of the pipes 710 is less than a shortest distance between the lower opening 551 of the first detection passage 550 and the top portions of the pipes 710. Assuming that the length and the width of the overall appearance of the portable respiratory system 1 are not greater than 95 mm and the height is not greater than 65 mm, the first length L1 can be approximately 13 mm and the second length L2 can be approximately 11 mm.

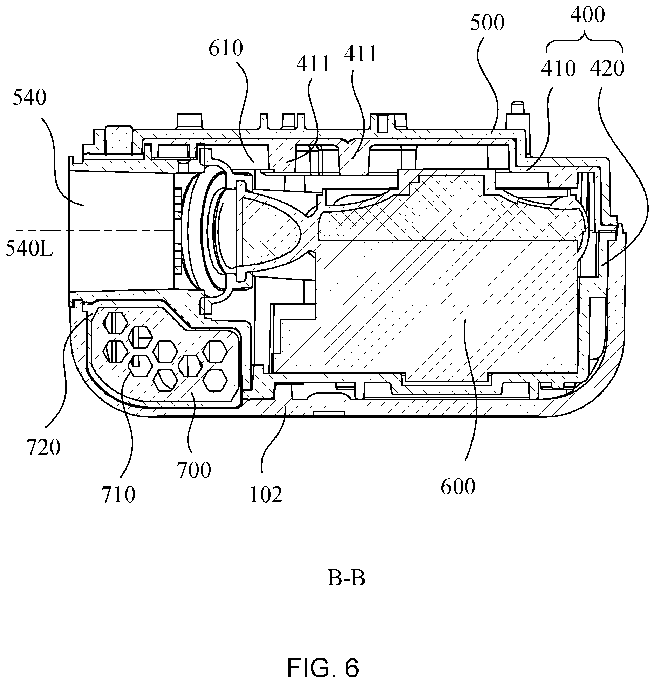

Again referring to FIG. 6, in this embodiment, the flow limiter 700 can be, for example, honeycomb flow channels, that is, each pipe 710 has a hexagonal cross-sectional area. The number and the cross-sectional area of the pipes 710 are associated with an area of a fluid allowed to pass through, and are designed according to the cross-sectional area and the shape of the lateral flow channel 520.

The area of the fluid allowed to pass through the flow limiter 700 (that is, a total area of through holes of the pipes 710) of this embodiment makes up about 25% to 30% of the cross-sectional area of the lateral flow channel 520. As an example, the cross-sectional area of the lateral flow channel 520 is approximately 336 mm², and the total area of the through holes of the pipes 710 (a total cross-sectional area allowing an airflow to pass through) is approximately 92.8 mm². The flow limiter 700 of this embodiment can include 4 to 12 pipes 710 having hexagonal cross-sectional areas, and each pipe 710 is a regular hexagon having an inscribed circle radius of 1.73 mm. It should be noted that the cross-sectional area of the pipe is not limited to the example above, and can also be other polygons.

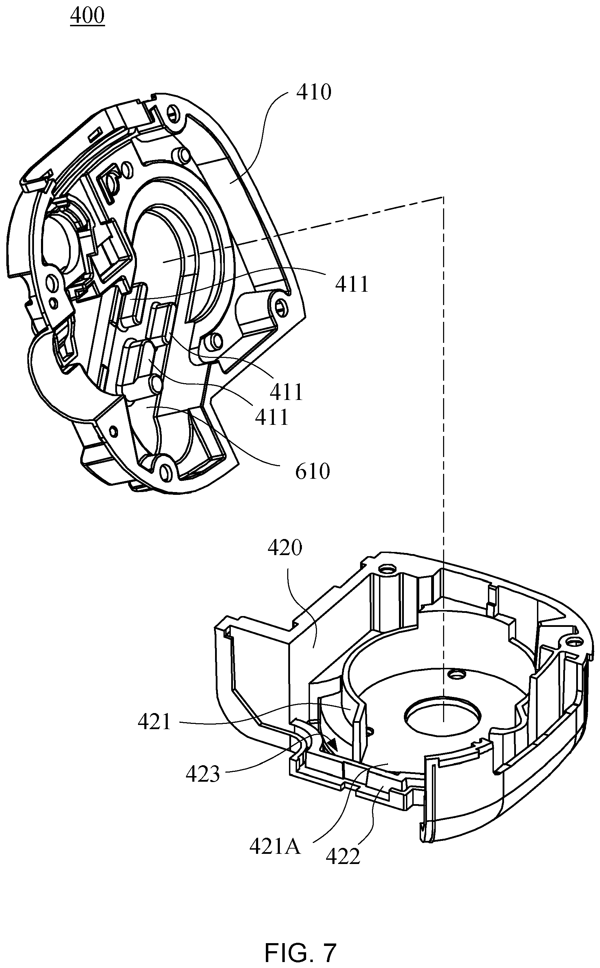

Referring to FIG. 7, FIG. 7 shows a perspective schematic diagram of an elastic member in a portable respiratory system according to this embodiment of the present invention.

The elastic member 400 can include an upper wind guide cover 410 and a lower shock absorbing cover 420. The upper wind guide cover 410 and the lower shock absorbing cover 420 can be made of a silicone material or other elastic materials so as to be elastic and flexible. The upper wind guide cover 410 and the blower 600 define an input flow channel 610. The input flow channel 610 is connected to the transmission flow channel 530 to guide the airflow transmitted from the transmission flow channel 530 into the blower 600. The upper wind guide cover 410 has multiple flow guide protrusions 411 protruding downward, so as to guide and rectify the airflow entering the input flow channel 610.

Again referring to FIG. 5(a) and FIG. 5(b), the rigid member 500 includes multiple protruding flow guide plates 511 and a spacing plate 512 in the air inlet flow channel 510. The multiple flow guide plates 511 separate a front section of the air inlet flow channel 510 into multiple air inlet channels 513, the spacing plate 512 is disposed on a middle section of the air inlet flow channel 510, and a bottom edge 512A of the spacing plate 512 is closer to a bottom portion of the housing 100 (a bottom shell wall of the lower shell 102) than a bottom edge 511A of the flow guide plates 511, so that a winding path along which the airflow flows is elongated and that it is less likely that internal noise of a motor be transmitted to the outside.

When the blower 600 is activated, a negative pressure is formed in the air inlet flow channel 510 to draw an airflow to enter the air inlet flow channel 510. Referring to FIG. 3 to FIG. 5, the airflow entering from the air inlet IN of the housing 100 is guided by the spacing plate 512 and the flow guide plates 511 and flows downward, passes through below the bottom edge 512A of the spacing plate 512, that is, a channel formed between the spacing plate 512 and the lower shock absorbing cover 420 of the elastic member 400, and flows toward the first opening 570 (FIG. 3 and FIG. 5(a)) of the rigid member 500, wherein the flow channel at this part is the air inlet flow channel 510. The airflow flows into the first opening 570 and enters the lateral flow channel 520.

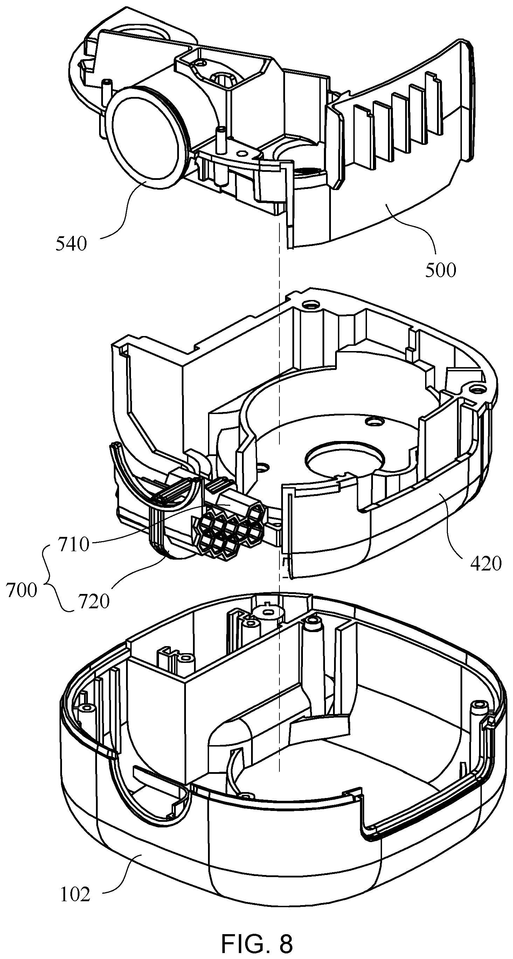

Referring to FIG. 2 and FIG. 8, FIG. 8 shows a partial exploded schematic diagram of a flow limiter, a rigid member, a lower shock absorbing cover and a lower shell of a portable respiratory system according to an embodiment of the present invention.

The flow limiter 700 is detachably or integrally disposed in the portable respiratory system 1. In this embodiment, the flow limiter 700 is designed to be detachably disposed in the lateral flow channel 520. The flow limiter 700 is not limited to being integrally formed with the rigid member 500 or the housing 100, but can also be a detachable discrete component.

The flow limiter 700 includes a sealing member 720. The sealing member 720 covers on the outside of the pipes 710 so as to fix all of the pipes 710, and position the flow limiter 700 between the air outlet flow channel 540 of the rigid member 500 and a bottom surface of the lower shell 102 of the housing 100. Moreover, airtightness is formed between the sealing member 720 and an outer wall of the air outlet flow channel 540, so that the airflow in the lateral flow channel 520 can pass only through the pipes 710 of the flow limiter 700. Meanwhile, the sealing member 720 further provides airtightness between a lower half of the air outlet flow channel 540 and the lower shell 102.

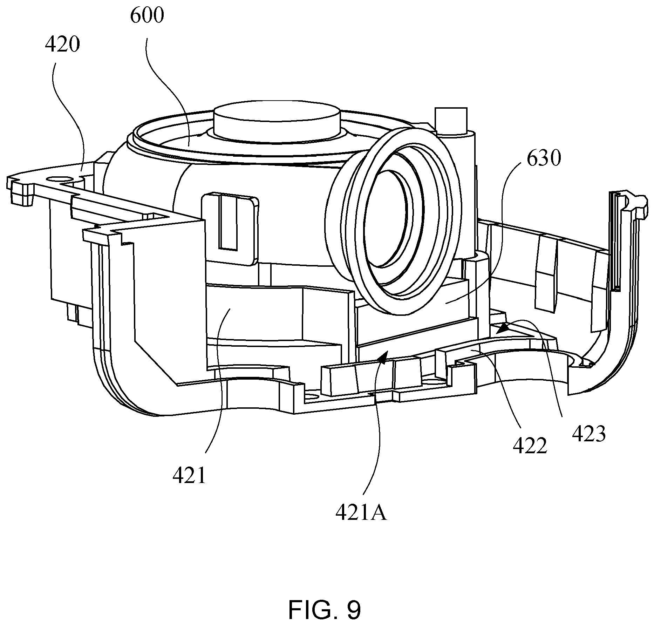

Referring to FIG. 7 and FIG. 9, FIG. 9 shows a schematic diagram of a blower and a lower shock absorbing cover in a portable respiratory system according to an embodiment of the present invention.

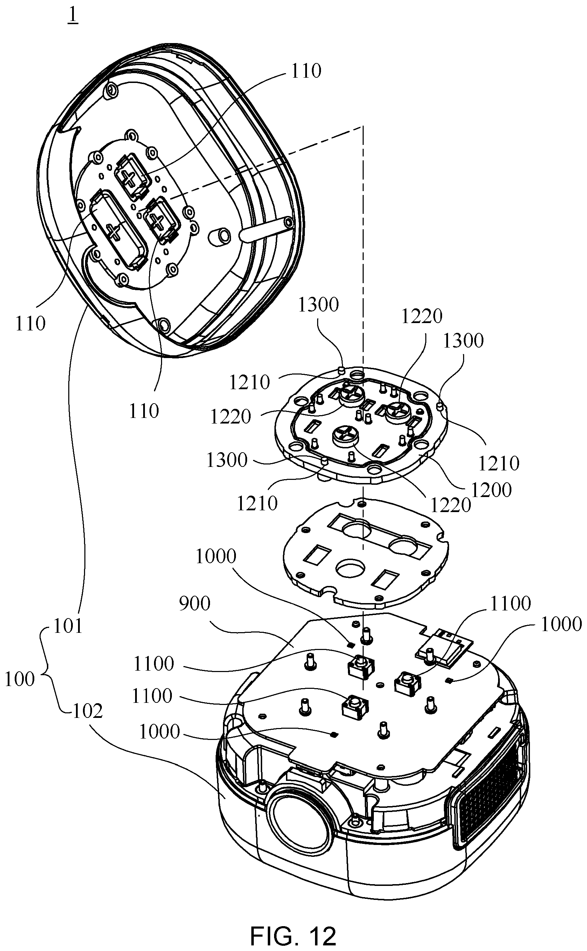

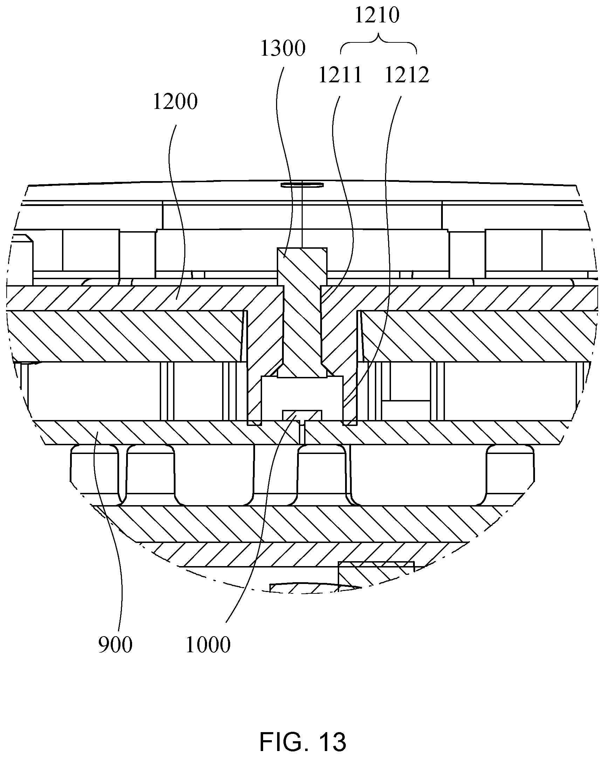

The lower shock absorbing cover 420 can include a positioning wall 421 and a water blocking wall 422 protruding upward. The positioning wall 421 covers from the side at least a portion of the blower 600, and the blower 600 is limited on a predetermined position by the positioning wall 421. As shown in FIG. 7 and FIG. 9, the extending positioning wall 421 defines the position for receiving the blower 600. Moreover, the positioning wall 421 can have a notch 421A to receive an electrical portion 630 of the blower 600. The electrical portion 630 can be a connection port and is electrically connected via a conducting line (not shown) to the circuit board 900 (referring to FIG. 12 and FIG. 13) located in the upper chamber, for the blower 600 to obtain power and an electrical signal provided to the blower 600 from the circuit board 900. The water blocking wall 422 is disposed on the periphery of the notch 421A, and is, for example, a rib structure extending and formed on an adjacent side of the notch 421A. In the event of moisture seeping into the housing 100, the water blocking wall 422 can further practice a function of blocking moisture from entering the blower 600. A recess 423 can further be disposed between the water blocking wall 422 and the notch 421A of the positioning wall 421, so as to further reduce the risk of water entering the blower 600 in case that a large amount of moisture enters and accumulates at this part. The positioning wall 421 and the water blocking wall 422 can be an integrally formed structure of the lower shock absorbing cover 420.

Generally speaking, the lower shock absorbing cover 420 can form a higher and almost seamless surrounding wall on at least left, rear and right sides of the blower 600 by an integrally formed structure, and then form a water blocking structure by surrounding the blower 600 with the rigid member 500 and the surrounding wall on the front side of the blower 600 by being assembled with the rigid member 500. Since the lower shock absorbing cover 420 and the rigid member 500 are assembled, there is a possibility of water seeping through an inevitable gap. Thus, with the lower shock absorbing cover 420 designed with the positioning wall 421 and the water blocking wall 422 along with the notch 421A, any liquid seeping from the side and the bottom into the blower 600 can be effectively prevented.

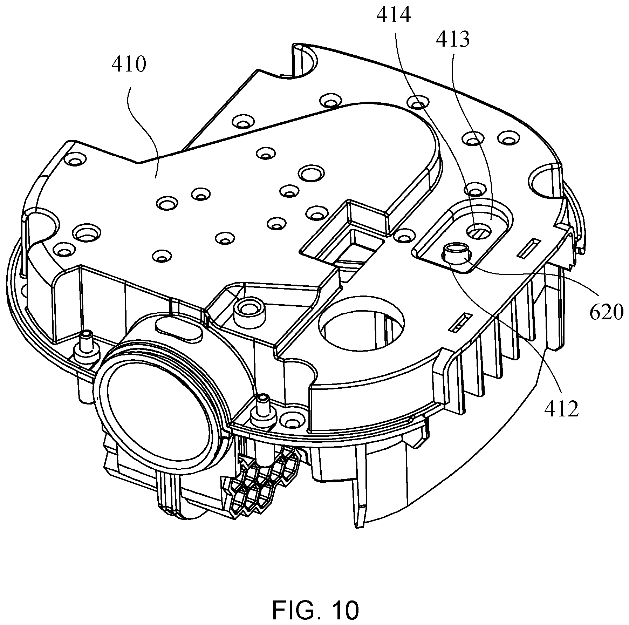

Refer to FIG. 10 showing a schematic diagram of a blower and an upper wind guide cover in a portable respiratory system according to an embodiment of the present invention.

The upper wind guide cover 410 can have an exhaust hole 412 which is operable for a heat exhaust pipe 620 of the blower 600 to dissipate heat energy. As shown in FIG. 10, the upper wind guide cover 410 has a convection groove 413 and a convection hole 414 formed near the exhaust hole 412. The convection hole 414 is in communication with the chamber wherein the convection groove 413 and the blower 600 are located, the convection groove 413 is formed as recessed from the upper wind guide cover 410, and the convection hole 414 passes through the upper wind guide cover 410 from a bottom of the convection groove 413. When the divider 200 and the upper wind guide cover 410 are assembled, a top surface of the convection groove 413 is covered by a bottom surface of the divider 200, and the convection groove 413 can then be in communication with the outside via only the exhaust hole 412 and the convection hole 414. The heat exhaust pipe 620 of the blower 600 extends into the convection groove 413 via the exhaust hole 412. A negative pressure is formed in a chamber where the blower 600 is located during the operation of the blower 600, and the negative pressure forms traction on the air in the convection groove 413 via the convection hole 414 to generate forced convection in the convection groove 413, so as to guide hot air in the heat exhaust pipe 620 after thermal exchange into the chamber where the blower 600 is located to form circulation. Once having entered the chamber where the blower 600 is located, the hot air is quickly discharged from the air outlet flow channel 540 along with the operation of the blower 600, and heat is thus prevented from accumulating in the blower 600.

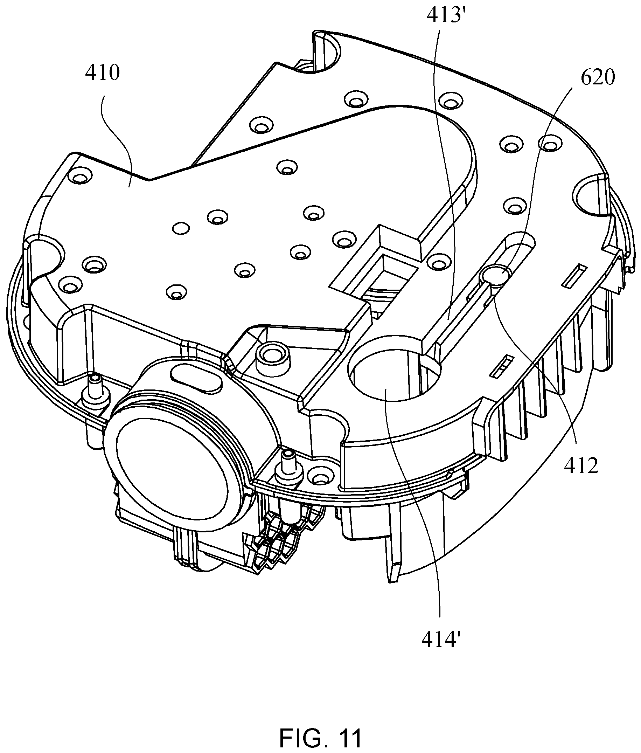

Refer to FIG. 11 showing a schematic diagram of a blower and an upper wind guide cover in a portable respiratory system according to another embodiment of the present invention.

As shown in FIG. 11, in this embodiment, a convection groove 413' of the upper wind guide cover 410 is formed as recessed from the upper wind guide cover 410 and extends at the upper wind guide cover 410, the exhaust hole 412 and a convection hole 414' individually pass through the upper wind guide cover 410, and the convection groove 413' is operable to be in communication with the exhaust hole 412 and the convection hole 414'. When the divider 200 and the upper wind guide cover 410 are assembled, a top surface of the convection groove 413' is covered by the bottom surface of the divider 200, so that the convection groove 413' can only communication with the outside via only the exhaust hole 412 and the convection hole 414'. The heat exhaust pipe 620 of the blower 600 is in communication with the convection groove 413', the convection hole 414' and the air inlet flow channel 510 by means of extending into the exhaust hole 412. Thus, during the operation of the blower 600, the negative pressure formed in the air inlet flow channel 510 can form traction on the air in the convection groove 413' based on the convection hole 414', further at the same time forming traction on the air in the heat exhaust pipe 620 of the blower 600 by the exhaust hole 412, so as to guide the hot air in the heat exhaust pipe 620 after thermal exchange to enter the air inlet flow channel 510 through the convection groove 413', and be added to an airflow that is about to enter the transmission flow channel 530. The hot air is quickly discharged from the air outlet flow channel 540 along with the operation of the blower 600, and thus heat is prevented from accumulating in the blower 600.

Referring to FIG. 12 and FIG. 13, FIG. 12 shows a partial exploded schematic diagram of a portable respiratory system according to an embodiment of the present invention, and FIG. 13 shows a cross-sectional schematic diagram of a portion of FIG. 12.

As shown in FIG. 12, the portable respiratory system 1 of this embodiment can further include the circuit board 900, a light emitting element 1000, a button element 1100, a shade hood 1200 and a light guide column 1300. The circuit board 900 is disposed in the upper chamber, and is sandwiched between the upper shell 101 and the divider 200. The flow sensor 300 is disposed on the circuit board 900.

The light emitting element 1000 and the button element 1100 are disposed on the circuit board 900. The light emitting element 1000 can be, for example, an LED element, and is operable to indicate a state or a control mode of the portable respiratory system. The button element 1100 is operable to be pressed to correspondingly issue an electrical signal, and the light emitting element 1000 is triggered to emit light based on a control command generated by a line and an electronic element on the circuit board 900. The shade hood 1200 covers the light emitting element 1000, and includes a through opening 1210. The light guide column 1300 is disposed in the through opening 1210, and is operable to guide light, so that the light emitted by the light emitting element 1000 can be gathered by the light guide column 1300 and guided to a predetermined position. As an example, the shade hood 1200 is made of a silicone material, and the light guide column 1300 can be made of a PC material or a PMMA material.

As shown in FIG. 13, in the shade hood 1200, the through opening 1210 has a first section 1211 and a second section 1212. The first section 1211 is operable to receive the light guide column 1300, the second section 1212 is operable to cover each light emitting element 1000, and an inner diameter of the second section 1212 is greater than an inner diameter of the first section 1211.

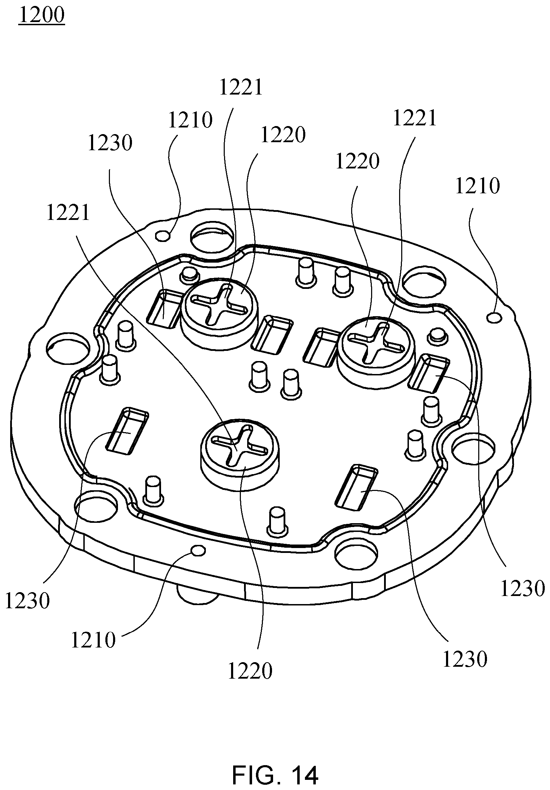

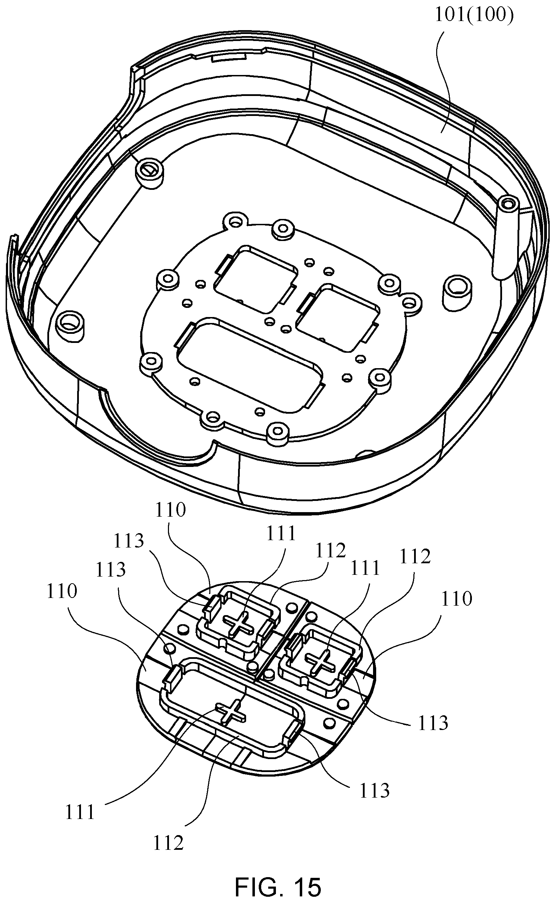

Referring to FIG. 14 and FIG. 15, FIG. 14 shows a schematic diagram of a shade hood in a portable respiratory system according to an embodiment of the present invention, and FIG. 15 shows a schematic diagram of an upper shell having a press member in a portable respiratory system according to an embodiment of the present invention.

As shown in FIG. 14 and FIG. 15, the shade hood 1200 further includes a protruding unit 1220, and a lower portion of the protruding unit 1220 corresponds to the button element 1100 on the circuit board 900. A press member 110 is disposed on an outer surface of the housing 100, and a top surface of the protruding unit 1220 has a slot 1221 for matching with a bottom surface structure 111 of the corresponding press member 110. By fitting the bottom surface structure 111 of the press member 110 with the slot 1221 of the protruding unit 1220, the press member 110 is prevented from swaying when being pressed. As an example, the slot 1221 is a cross positioning slot, and the bottom surface structure 111 is a cross positioning rib matching with the cross positioning slot. The cross matching structures provide positioning in four directions (front, rear, left and right), and are more stable in comparison with protruding/recessed matching structures in merely a linear form.

A bottom surface of the press member 110 can have a support wall 112, which is disposed around the bottom surface structure 111. When the press member 110 is pressed downward, the support wall 112 at the same time provides support at a surface of the shade hood 1200, hence increasing a contact surface area between the press member 110 and the shade hood 1200 and accordingly enhancing the stability when the press member 110 is pressed downward.

The bottom surface of the press member 110 can further have a stop portion 113, and the shade hood 1200 has a recess 1230 for correspondingly accommodating the stop portion 113, accordingly further enhancing the stability when the press member 110 is pressed downward. Meanwhile, the press member 110 is movable downward relative to the housing 100 and can be a discrete structure separate from housing 100. However, with the slot 1221 and the bottom surface structure 111 of the cross matching structures as well as the stop portion 113 and the recess 1230, the level of swaying of the press member 110 relative to the housing 100 can be reduced.

The portable respiratory system in the embodiments of the present invention features miniaturization, lightweight and easy portability, and forms an arrangement of an accommodation space and flow channels by structures of the divider, the elastic member, the rigid member and the flow limiter, further achieving a compact design. Meanwhile, the portable respiratory system has a flow monitoring function, and is able to regulate an amount of airflow or perform control of different air supply modes according to user requirements, thus improving comfort and experience of user of the respiratory system and at the same time promoting implementation of better therapeutic effects.

The present disclosure is illustrated by various aspects and embodiments. However, persons skilled in the art understand that the various aspects and embodiments are illustrative rather than restrictive of the scope of the present disclosure. After perusing this specification, persons skilled in the art may come up with other aspects and embodiments without departing from the scope of the present disclosure. All equivalent variations and replacements of the aspects and the embodiments must fall within the scope of the present disclosure. Therefore, the scope of the protection of rights of the present disclosure shall be defined by the appended claims.

## Claims

1. A portable respiratory system (1), comprising:
a housing (100);
a divider (200), disposed in the housing (100) and partitioning an internal space of the housing (100) into an upper chamber and a lower chamber;
a flow sensor (300), disposed in the upper chamber;
an elastic member (400), disposed in the lower chamber;
a rigid member (500), fixed in the lower chamber by the elastic member (400) and the housing (100), defining an air inlet flow channel (513), a lateral flow channel (520), a transmission flow channel (530) and an air outlet flow channel (540), wherein the lateral flow channel (520) is connected between the air inlet flow channel (513) and the transmission flow channel (530);
a blower (600), disposed in the elastic member (400), operable to receive an airflow from the transmission flow channel (530) to generate a positive pressure airflow, and transmit the airflow to the air outlet flow channel (540); and
a flow limiter (700), disposed in the lateral flow channel (520), the flow limiter (700) comprising a plurality of pipes (710) extending along the lateral flow channel (520);
wherein the flow limiter (700) is disposed below the air outlet flow channel (540) of the rigid member (500),
wherein the rigid member (500) further comprises a first detection passage (550) and a second detection passage (560), the first detection passage (550) is located in an upstream (700A) of the flow limiter (700), the second detection passage (560) is located in a downstream (700B) of the flow limiter (700), and the first detection passage (550) and the second detection passage (560) are individually connected to the flow sensor (300).

2. The portable respiratory system according to claim 1, wherein each of the first detection passage (550) and the second detection passage (560) has a lower opening (551, 561), and the two lower openings (551, 561) are higher than an upper edge (710A) of a highest one of the pipes (710).

3. The portable respiratory system according to claim 2, wherein the lower opening (551) of the first detection passage (550) and top portions of the pipes (710) define a first buffer space (S1) in between, the lower opening (561) of the second detection passage (560) and the top portions of the pipes (710) define a second buffer space (S2) in between, and airflow speeds in the first buffer space (S1) and the second buffer space (S2) are lower than an airflow speed at an opening of each of the pipes (710).

4. The portable respiratory system according to claim 3, wherein the first buffer space (S1) and the second buffer space (S2) are in non-symmetrical arrangement in between with respect to a central axis of the air outlet flow channel (540).

5. The portable respiratory system according to claim 3, wherein each of the pipes (710) extends from right below a central axis of the air outlet flow channel (540) in a direction away from the air inlet flow channel (513) by a first length (L1) and extends in a direction toward the air inlet flow channel (513) by a second length (L2), and the first length (L1) is greater than the second length (L2).

6. The portable respiratory system according to claim 2, wherein a vertical distance between the two lower openings and the upper edge (710A) of the highest one of the pipes (710) is a first distance (D1), which is greater than or equal to a half of an inner diameter of the pipe (710).

7. The portable respiratory system according to claim 1, wherein the first detection passage (550) and the second detection passage (560) protrude downward into the lateral flow channel (520).

8. The portable respiratory system according to claim 1, wherein the flow limiter (700) comprises a sealing member (720), the sealing member (720) covers on an outside of the pipes (710) so as to fix the pipes (710), and the sealing member (720) and an outer wall of the air outlet flow channel (540) form airtightness in between.

9. The portable respiratory system according to claim 1, wherein the flow limiter (700) is detachably disposed in the lateral flow channel (520).

10. The portable respiratory system according to claim 1, wherein the elastic member (400) comprises an upper wind guide cover (410) and a lower shock absorbing cover (420), the upper wind guide cover (410) and the blower (600) define an input flow channel (610), the input flow channel (610) is connected to the transmission flow channel (530) to guide an airflow to flow into the blower (600), and the upper wind guide cover (410) has a plurality of flow guide protrusions (411) protruding downward.

11. The portable respiratory system according to claim 10, wherein the upper wind guide cover (410) has an exhaust hole (412) which is operable for a heat exhaust pipe (620) of the blower (600) to dissipate heat energy.

12. The portable respiratory system according to claim 1, wherein the rigid member (500) comprises a plurality of protruding flow guide plates (511) and a spacing plate (512) at the air inlet flow channel (513), the flow guide plates (511) separate a front section of the air inlet flow channel (513) into a plurality of air inlet channels (513), the spacing plate (512) is disposed at a middle section of the air inlet flow channel (513), and a bottom edge of the spacing plate (512) is closer to a bottom portion of the housing (100) than bottom edges of the flow guide plates (511).

13. The portable respiratory system according to claim 10, wherein the lower shock absorbing cover (420) comprises a positioning wall (421) and a water blocking wall (422) protruding upward, the positioning wall covers at least a portion of the blower (600) and has a notch (421A) to receive an electrical portion (630) of the blower (600), and the water blocking wall (422) is disposed on the periphery of the notch (421A).

## Patentansprüche

1. Tragbares Atemschutzsystem (1), umfassend:
ein Gehäuse (100),
einen Trenner (200), der im Gehäuse (100) angeordnet ist und den Innenraum des Gehäuses (100) in eine obere Kammer und eine untere Kammer unterteilt,
einen Durchflusssensor (300), der in der oberen Kammer angeordnet ist,
ein elastisches Element (400), das in der unteren Kammer angeordnet ist,
ein starres Element (500), das durch das elastische Element (400) und das Gehäuse (100) in der unteren Kammer befestigt ist und einen Lufteinlassströmungskanal (513), einen seitlichen Strömungskanal (520), einen Übertragungsströmungskanal (530) und einen Luftauslassströmungskanal (540) definiert, wobei der seitliche Strömungskanal (520) zwischen den Lufteinlassströmungskanal (513) und den Übertragungsströmungskanal (530) angeschlossen ist,
ein Gebläse (600), das im elastischen Element (400) angeordnet ist und betätigt werden kann, um einen Luftstrom aus dem Übertragungsströmungskanal (530) aufzunehmen, um einen Überdruckluftstrom zu erzeugen, und den Luftstrom an den Luftauslassströmungskanal (540) weiterzuleiten, und
einen Strömungsbegrenzer (700), der im seitlichen Strömungskanal (520) angeordnet ist, wobei der Strömungsbegrenzer (700) eine Vielzahl von Rohren (710) umfasst, die sich entlang des seitlichen Strömungskanals (520) erstrecken,
wobei der Strömungsbegrenzer (700) unterhalb des Luftauslassströmungskanals (540) des starren Elements (500) angeordnet ist,
wobei das starre Element (500) ferner einen ersten Detektionsdurchgang (550) und einen zweiten Detektionsdurchgang (560) umfasst, wobei sich der erste Detektionsdurchgang (550) stromaufwärts (700A) des Strömungsbegrenzers (700) befindet, sich der zweite Detektionsdurchgang (560) stromabwärts (700B) des Strömungsbegrenzers (700) befindet und der erste Detektionsdurchgang (550) und der zweite Detektionsdurchgang (560) jeweils separat mit dem Durchflusssensor (300) verbunden sind.

2. Tragbares Atemschutzsystem nach Anspruch 1, wobei sowohl der erste Detektionsdurchgang (550) als auch der zweite Detektionsdurchgang (560) eine untere Öffnung (551, 561) aufweisen und die beiden unteren Öffnungen (551, 561) höher liegen als eine Oberkante (710A) des höchsten der Rohre (710).

3. Tragbares Atemschutzsystem nach Anspruch 2, wobei die untere Öffnung (551) des ersten Detektionsdurchgangs (550) und die oberen Abschnitte der Rohre (710) einen ersten Pufferraum (S1) dazwischen definieren, die untere Öffnung (561) des zweiten Detektionsdurchgangs (560) und die oberen Abschnitte der Rohre (710) einen zweiten Pufferraum (S2) dazwischen definieren und die Luftströmungsgeschwindigkeiten im ersten Pufferraum (S1) und im zweiten Pufferraum (S2) niedriger sind als die Luftströmungsgeschwindigkeit an einer Öffnung jedes der Rohre (710).

4. Tragbares Atemschutzsystem nach Anspruch 3, wobei der erste Pufferraum (S1) und der zweite Pufferraum (S2) in Bezug auf eine Mittelachse des Luftauslassströmungskanals (540) asymmetrisch angeordnet sind.

5. Tragbares Atemschutzsystem nach Anspruch 3, wobei sich jedes der Rohre (710) von direkt unterhalb einer Mittelachse des Luftauslassströmungskanals (540) in einer Richtung weg vom Lufteinlassströmungskanal (513) um eine erste Länge (L1) erstreckt und sich in einer Richtung zum Lufteinlassströmungskanal (513) hin um eine zweite Länge (L2) erstreckt, wobei die erste Länge (L1) größer als die zweite Länge (L2) ist.

6. Tragbares Atemschutzsystem nach Anspruch 2, wobei der vertikale Abstand zwischen den beiden unteren Öffnungen und der Oberkante (710A) des höchsten der Rohre (710) ein erster Abstand (D1) ist, der größer oder gleich der Hälfte des Innendurchmessers des Rohrs (710) ist.

7. Tragbares Atemschutzsystem nach Anspruch 1, wobei der erste Detektionsdurchgang (550) und der zweite Detektionsdurchgang (560) nach unten in den seitlichen Strömungskanal (520) hineinragen.

8. Tragbares Atemschutzsystem nach Anspruch 1, wobei der Strömungsbegrenzer (700) ein Dichtungselement (720) umfasst, das Dichtungselement (720) die Außenseite der Rohre (710) abdeckt, um die Rohre (710) zu fixieren, und das Dichtungselement (720) und eine Außenwand des Luftauslassströmungskanals (540) dazwischen eine Luftdichtheit bilden.

9. Tragbares Atemschutzsystem nach Anspruch 1, wobei der Strömungsbegrenzer (700) abnehmbar im seitlichen Strömungskanal (520) angeordnet ist.

10. Tragbares Atemschutzsystem nach Anspruch 1, wobei das elastische Element (400) eine obere Windleitungsabdeckung (410) und eine untere stoßdämpfende Abdeckung (420) umfasst, wobei die obere Windleitungsabdeckung (410) und das Gebläse (600) einen Einlassströmungskanal (610) bilden, der Einlassströmungskanal (610) mit dem Übertragungsströmungskanal (530) verbunden ist, um einen Luftstrom in das Gebläse (600) zu leiten, und die obere Windleitungsabdeckung (410) mehrere nach unten vorstehende Strömungsleitungsvorsprünge (411) aufweist.

11. Tragbares Atemschutzsystem nach Anspruch 10, wobei die obere Windleitungsabdeckung (410) ein Auslassloch (412) aufweist, das für ein Wärmeabzugsrohr (620) des Gebläses (600) zur Ableitung von Wärmeenergie betätigbar ist.

12. Tragbares Atemschutzsystem nach Anspruch 1, wobei das starre Element (500) mehrere vorstehende Strömungsleitplatten (511) und eine Abstandshalterplatte (512) am Lufteinlassströmungskanal (513) umfasst, wobei die Strömungsleitplatten (511) einen vorderen Abschnitt des Lufteinlassströmungskanals (513) in mehrere Lufteinlasskanäle trennen, die Abstandshalterplatte (512) in einem mittleren Abschnitt des Lufteinlassströmungskanals (513) angeordnet ist und eine Unterkante der Abstandshalterplatte (512) näher an einem unteren Abschnitt des Gehäuses (100) liegt als die Unterkanten der Strömungsleitplatten (511).

13. Tragbares Atemschutzsystem nach Anspruch 10, wobei die untere stoßdämpfende Abdeckung (420) eine Positionierungswand (421) und eine nach oben vorstehende wasserblockierende Wand (422) umfasst, wobei die Positionierungswand mindestens einen Teil des Gebläses (600) abdeckt und eine Aussparung (421A) zur Aufnahme eines elektrischen Teils (630) des Gebläses (600) aufweist und die wasserblockierende Wand (422) am Umfang der Aussparung (421A) angeordnet ist.

## Revendications

1. Un système respiratoire portable (1) comprenant:
un boîtier (100);
une cloison (200) disposée dans le boîtier (100) et divisant l'espace interne du boîtier (100) en une chambre supérieure et une chambre inférieure;
un capteur de débit (300) disposé dans la chambre supérieure;
un élément élastique (400) disposé dans la chambre inférieure;
un élément rigide (500) fixé dans la chambre inférieure par l'élément élastique (400) et le boîtier (100), définissant un canal d'entrée d'air (513), un canal d'écoulement latéral (520), un canal de transmission (530) et un canal d'évacuation d'air (540), dans lequel le canal d'écoulement latéral (520) est relié au canal d'entrée d'air (513) et au canal de transmission (530) ;
une soufflante(600), disposée dans l'élément élastique (400), conçu pour recevoir un flux d'air provenant du canal de transmission (530) afin de générer un flux d'air sous pression positive et de transmettre ce flux d'air au canal d'évacuation d'air (540) ; et
un limiteur de débit (700), disposé dans le canal d'écoulement latéral (520), ce limiteur de débit (700) comprenant une pluralité de tuyaux (710) s'étendant le long du canal d'écoulement latéral (520);
Dans lequel le limiteur de débit (700) est disposé sous le canal d'évacuation d'air (540) de l'élément rigide (500),
dans lequel cet élément rigide (500) comprend en outre un premier passage de détection (550) et un second passage de détection (560), le premier passage de détection (550) est situé en amont (700A) du limiteur de débit (700), le second passage de détection (560) est situé en aval (700B) du limiteur de débit (700), et le premier passage de détection (550) et le second passage de détection (560) sont reliés individuellement au capteur de débit (300).

2. Le système respiratoire portable selon la revendication 1, dans lequel chacun du premier passage de détection (550) et du second passage de détection (560) présente une ouverture inférieure (551, 561), ces deux ouvertures inférieures (551, 561) sont plus hautes que le bord supérieur (710A) du plus haut des tuyaux (710).

3. Le système respiratoire portable selon la revendication 2, dans lequel l'ouverture inférieure (551) du premier passage de détection (550) et les parties supérieures des tuyaux (710) définissent un premier espace tampon (S1) entre eux, l'ouverture inférieure (561) du deuxième passage de détection (560) et les parties supérieures des tuyaux (710) définissent un deuxième espace tampon (S2) entre eux, et les vitesses d'écoulement d'air dans le premier espace tampon (S1) et le deuxième espace tampon (S2) sont inférieures à la vitesse d'écoulement d'air à l'ouverture de chacun des tuyaux (710).

4. Le système respiratoire portable selon la revendication 3, dans lequel le premier espace tampon (S1) et le second espace tampon (S2) sont disposés de manière asymétrique l'un par rapport à l'autre par rapport à l'axe central du canal d'évacuation d'air (540).

5. Le système respiratoire portable selon la revendication 3, dans lequel chacun des tuyaux (710) s'étend, depuis juste en dessous d'un axe central du canal d'évacuation d'air (540) dans une direction s'éloignant du canal d'entrée d'air (513) sur une première longueur (L1) et s'étend dans une direction vers le canal d'entrée d'air (513) sur une deuxième longueur (L2), et la première longueur (L1) est supérieure à la deuxième longueur (L2).

6. Le système respiratoire portable selon la revendication 2, dans lequel la distance verticale entre les deux ouvertures inférieures et le bord supérieur (710A) du tuyau (710) le plus haut est une première distance (D1) supérieure ou égale à la moitié du diamètre intérieur du tuyau (710).

7. Le système respiratoire portable selon la revendication 1, dans lequel le premier passage de détection (550) et le second passage de détection (560) font saillie vers le bas dans le canal d'écoulement latéral (520).

8. Le système respiratoire portable selon la revendication 1, dans lequel le limiteur de débit (700) comprend un élément d'étanchéité (720), l'élément d'étanchéité (720) recouvre l'extérieur des tuyaux (710) de manière à fixer les tuyaux (710), et l'élément d'étanchéité (720) et la paroi extérieure du canal d'évacuation d'air (540) forment une étanchéité entre eux.

9. Le système respiratoire portable selon la revendication 1, dans lequel le limiteur de débit (700) est disposé de manière amovible dans le canal d'écoulement latéral (520).

10. Le système respiratoire portable selon la revendication 1, dans lequel l'élément élastique (400) comprend un couvercle supérieur de guidage d'air (410) et un couvercle inférieur amortisseur (420), le couvercle supérieur de guidage d'air (410) et la soufflante(600) définissent un canal d'alimentation en air (610), le canal d'alimentation en air (610) est relié au canal de transmission (530) afin de guider un flux d'air vers la soufflante(600), et le couvercle supérieur de guidage d'air (410) présente plusieurs protubérances de guidage d'air (411) orientées vers le bas.

11. Le système respiratoire portable selon la revendication 10, dans lequel le couvercle supérieur de guidage d'air (410) comporte un orifice d'évacuation (412) permettant à un tuyau d'évacuation de chaleur (620) de la soufflante(600) de dissiper l'énergie thermique.

12. Le système respiratoire portable selon la revendication 1, dans lequel l'élément rigide (500) comprend une pluralité de plaques de guidage de flux saillantes (511) et une plaque d'espacement (512) au niveau du canal d'entrée d'air (513), les plaques de guidage de flux (511) séparent une section avant du canal d'entrée d'air (513) en une pluralité de passages d'entrée d'air, la plaque d'espacement (512) est disposée dans une section médiane du canal d'entrée d'air (513), et un bord inférieur de la plaque d'espacement (512) est plus proche d'une partie inférieure du boîtier (100) que les bords inférieurs des plaques de guidage de flux (511).

13. Le système respiratoire portable selon la revendication 10, dans lequel le couvercle inférieur absorbant les chocs (420) comprend une paroi de positionnement (421) et une paroi de blocage d'eau (422) faisant saillie vers le haut, la paroi de positionnement recouvrant au moins une partie de la soufflante(600) et comportant une encoche (421A) destinée à recevoir une partie électrique (630) de la soufflante(600), et la paroi de blocage d'eau (422) est disposée sur la périphérie de l'encoche (421A).
